# EUROPEAN PATENT SPECIFICATION

(11) **EP 2 313 146 B1**
(45) Date of publication and mention of the grant of the patent: **01.05.2019**
(21) Application number: 09798272.2
(22) Date of filing: 13.07.2009
(51) Int. Cl.: A61B 17/22

(54) **DEVICES FOR THE TREATMENT OF VASCULAR ANEURYSM**
VORRICHTUNGEN ZUR BEHANDLUNG VON VASKULÄREM ANEURYSMA
DISPOSITIFS POUR LE TRAITEMENT D'UN ANÉVRYSME VASCULAIRE

(30) Priority: 15.07.2008 US 173726
(43) Date of publication of application: 27.04.2011
(73) Proprietor: Vatrix Medical, Inc., Maple Grove, MN 55369 (US)
(72) Inventor: OGLE, Matthew, F., Fitchburg WI 53711 (US); ISENBURG, Jason, C., Victoria MN 55386 (US)
(74) Representative: dompatent von Kreisler Selting Werner - Partnerschaft von Patent- und Rechtsanwälten mbB
(86) International application number: PCT/US2009/004044
(87) International publication number: WO 2010/008513

(56) References cited:
- WO-A1-98/26832
- US-A- 5 342 306
- US-A- 5 376 114
- US-A- 5 514 092
- US-A- 5 558 642
- US-A- 5 645 540
- US-A1- 2001 018 569
- US-A1- 2003 078 544
- US-A1- 2005 085 770
- US-A1- 2006 041 269

## Description

### Field of the Invention

The invention, in general, is related to the treatment of vascular aneurysms using a device for the delivery of selected stabilization compositions for localized delivery in the vicinity of the aneurysm. The invention is further related to devices that can isolate a volume in the vicinity of the aneurysm to perform treatment. The blood in the isolated volume around aneurysm can be aspirated before the stabilization composition is delivery by the device to the vicinity of the aneurysm to reduce dilution of the composition.

### Background

Aneurysms are degenerative diseases characterized by destruction of arterial architecture and subsequent dilatation of the blood vessel that may eventually lead to fatal ruptures. Some common locations for aneurysms include the abdominal aorta (abdominal aortic aneurysm, AAA), thoracic aorta, and brain arteries. In addition, peripheral aneurysms of the leg, namely the popliteal and femoral arteries are prevalent locations of this vascular pathology. The occurrence of such peripheral aneurysms appears to be strongly associated with the presence of aneurysms in other locations, as it has been estimated that 30 to 60% of peripheral aneurysm patients also have an AAA.

Aneurysms grow over a period of years and pose great risks to health. Aneurysms have the potential to dissect or rupture, causing massive bleeding, stroke, and hemorrhagic shock, which can be fatal in more than 80% of cases. AAAs are a serious health concern, specifically for the aging population, being among the top ten causes of death for patients older than 50. The estimated incidence for abdominal aortic aneurysm is about 50 in every 100,000 persons per year. Approximately 60,000 operations are performed each year in the U.S. for AAAs alone. In children, AAAs can result from blunt abdominal injury or from Marfan's syndrome, a defect in elastic fiber formation in walls of major arteries, such as the aorta.

Aneurysms can be caused by any of a large class of degenerative diseases and pathologies including atherosclerotic disease, defects in arterial components, genetic susceptibilities, and high blood pressure, among others, and can develop silently over a period of years. The hallmarks of aneurysms include enzymatic degradation of vascular structural proteins such as elastin, inflammatory infiltrates, calcification, and eventual overall destruction of the vascular architecture.

Current methods of treatment for diagnosed aneurysms are generally limited to invasive surgical techniques. After initial diagnosis of a small aneurysm, the most common medical approach is to follow up the development of the aneurysm and after reaching a predetermined size (e.g., about 5 cm in diameter), surgical treatment is applied. Current surgical treatments generally are limited to either an endovascular stent graft repair or optionally complete replacement of the diseased vessel with a vascular graft. While such surgical treatments can save lives and improve quality of life for those suffering aneurysm, dangers beyond those of the surgery itself still exist for the patient due to possible post-surgery complications (e.g., neurological injuries, bleeding, or stroke) as well as device-related complications (e.g., thrombosis, leakage, or failure). Moreover, depending upon the location or anatomy of the aneurysm, the danger of an invasive surgical procedure may outweigh the possible benefits of the procedure, for instance in the case of an aneurysm deep in the brain, leaving the sufferer with very little in the way of treatment options. Moreover, surgical treatments may not always provide a permanent solution, as vascular grafts can loosen and dislodge should the aneurysm progress following the corrective surgery. Generally, most of the current treatment options for aneurysm are mechanical bridges. For some patients, the particular nature of the aneurysm or the condition of the patient makes the patient unsuitable for graft repair.

Aneurysm is not the only condition for which enzymatic degradation of structural proteins is a hallmark. Other conditions in which structural protein degradation appears to play a key role include Marfan syndrome, supravalvular aortic stenosis, and chronic obstructive pulmonary disease (COPD). For those afflicted, such conditions lead to, at the very least, a lowered quality of life and often, premature death.

Document US 2005/0085770 A1, discloses a device for observing and treating body passages. The device is composed of: a first catheter dimensioned to be insertable into the body passage and having a lateral wall, a proximal end and a distal end; and a first balloon carried by the first catheter and extending outwardly from the lateral wall. The first catheter is provided internally with not more than three fluid conducting passages, including: a blood bypass flow passage extending at least from a first point located between the first balloon and the proximal end to a second point at the distal end and communicating at the first point with a region surrounding the first catheter; a balloon inflation passage communicating with the first balloon; and a delivery/aspiration passage opening at the lateral wall at a location between the first point and the first balloon.

Documents WO 98/26832; US 5,514,092; and US 2006/0041269 disclose alternative devices for treating isolated volumes in blood vessels.

### Summary of the Invention

In one aspect, a device for treating an isolated volume in a blood vessel, according to independent claim 1, is disclosed. Further aspects of the invention are defined in the dependent claims.

The device comprises a shaft and a sealing element. The shaft has a proximal end, a distal end and at least one lumen extending from at or near the proximal end to at or near the distal end. In one embodiment, the lumen connects with a port at its proximal end. The sealing element comprises an extendable element and a fluid exchange portion comprising one or more exchange ports in fluid communication with the lumen of the shaft. The extendable element has a lower profile configuration and an extended configuration having a shape that pushes against the wall of the vessel to form the isolated volume within the vessel with the exchange port of the fluid exchange portion comprising a flow channel configured for the exchange of fluid between the isolated volume and the lumen.

In one embodiment, the sealing element of the device when deployed in the extended configuration comprises a channel to allow blood flow past the isolated volume when the extendable element contacts the vessel wall. In one embodiment, the shaft of the device further comprises a rapid exchange guidewire lumen with a port at the distal end of the shaft.

In yet another embodiment, the extendable element of the device comprises an actuation element that controls the transition of the extendable element between the lower profile configuration and the extended configuration. In one embodiment, the fluid exchange portion of the device is on the shaft of the device comprising one or more exchange ports in fluid communication with the isolated volume. In one embodiment, the exchange port of the device further comprises a tubular conduit displaced from the shaft having an opening that is in fluid communication with the isolated volume. In another embodiment, the fluid exchange portion comprises a side channel that is a portion of the lumen having an opening on the side of the sealing element of the device. In one embodiment, the device further comprises a micro-catheter placed inside the side channel and the lumen to go through the opening on the side of the sealing element to access the isolated volume. In one embodiment, the exchange ports comprise a liquid permeable structure. In one embodiment, the device further comprises an aspiration apparatus operably connected to the port to one of the lumens of the shaft. In one embodiment, the aspiration apparatus comprises a syringe. In one embodiment, the device further comprises a delivery element operably connected to the port to one of the lumens of the shaft. The delivery element comprises a stabilizing liquid that reacts with vessel tissue to stabilize the tissue.

### Brief Description of the Drawings

Figure 1 is a schematic side view of a rapid exchange delivery device.
Figure 2 is a sectional view of the shaft of the delivery device of Fig. 1.
Figure 3 is a schematic view of the extended configuration of an embodiment of a delivery device.
Figure 4 is a sectional view of the extendable element of the device of Fig. 3.
Figure 5 is a schematic side view of the device of Fig. 3 deployed in the vicinity of an aneurysm.
Figure 6 is a schematic front view of the extended configuration of one embodiment of a delivery device.
Figure 7 is a schematic back view of the device of Fig. 6 deployed in the vicinity of an aneurysm.
Figure 8 is a schematic view of the extended configuration of one embodiment of a delivery device with a self extending support.
Figure 9 is a schematic side view of the extended configuration of another embodiment of a delivery device with a self extending support.
Figure 10 is a sectional view of the extendable element of the device of Fig. 9.
Figure 11 is a schematic view of the extended configuration of one embodiment of a delivery device with a self extending support.
Figure 12 is a schematic side view of the device of Fig. 11.
Figure 13 is a schematic view of the extended configuration of one embodiment of a delivery device with an actuation element.
Figure 14 is a schematic diagram of a device being directed to the vicinity of an aneurysm inside a vessel.
Figure 15 is the device of Fig. 14 being placed near aneurysm in a lower profile configuration.
Figure 16 is the device of Fig. 15 being deployed in an extended configuration to isolate a volume in the vicinity of the aneurysm.
Figure 17 is the deployed device of Fig. 16 aspirating fluid from the isolated volume in the vicinity of the aneurysm.
Figure 18 is the deployed device of Fig. 16 delivering an effective amount of a therapeutic composition into the isolated volume in the vicinity of the aneurysm.
Figure 19 is a schematic diagram of the device of Fig. 14 in the lower profile configuration being retrieved from the vessel.
Figure 20 is a schematic side view of the extended configuration of one embodiment of a delivery device, according to the invention, with a side channel on the shaft of the device.
Figure 21 is the device of Fig. 20 being directed to the vicinity of an aneurysm with a micro catheter placed inside the side channel of the device.

### Detailed Description of Preferred Embodiments

The devices described herein provide for the isolation of a volume around a selected portion of a blood vessel through a less invasive procedure that accesses the region of the blood vessel through the patient's vasculature or other vessel. The device further provides for the delivery of a therapeutic composition to an aneurysm or other vessel condition to the isolated volume such that systemic contact with the therapeutic composition and the dilution of the therapeutic composition can be significantly reduced or eliminated. In some embodiments, the approaches described herein provide treatment of an aneurysm with intravascular approach using a device to optionally aspirate blood around the aneurysm followed by the delivery of a stabilizing agent to the site of the aneurysm, and the blood vessel at the aneurysm can be thereby stabilized and/or subject to reduced further degradation of the vessel architecture supported by structural proteins, e.g. elastin. The isolated volume therefore, serves the purpose of improving the efficacy of the stabilizing agent. For appropriate embodiments, the stabilization agent maybe embedded in and/or associated with a delivery composition, such as a pluronic hydrogel and/or polymeric nanoparticles. In some embodiments, blood flow can be maintained past the isolated volume in the vessel through a flow channel designed to by-pass the isolated volume. While the description herein focuses on aortic aneurysms and cerebral aneurysm, the treatment approaches described herein can be generalized to other aneurysms as well as other vessel defects and diseases based on the teachings herein. In general, connective tissue targeted with the device can be stabilized so as to be less susceptible to protein degradation that can be brought about due to any of a variety of mechanisms and/or conditions including, for example, those associated with aneurysm, atherosclerotic disease, genetic susceptibilities, blunt force injury, Marfan's syndrome, and the like.

Connective tissue is the framework upon which the other types of tissue, i.e., epithelial, muscle, and nerve tissues, are supported. Connective tissue generally comprises individual cells not directly attached to one another and held within the extracellular matrix. The extracellular matrix, in turn, comprises compositions excreted by specific cells with specific mechanical properties, which include, for example, fibrous components such as collagen fibers and elastin fibers. Connective tissue can assume widely divergent architectures. Blood vessels generally involve connective tissue, for example, with a thin layer of endothelial cells lining the blood vessel.

At an aneurysm, blood vessels exhibit degradation of the tissue. Due to the blood pressure in the vessel, as the tissue of blood vessel weakens, the vessel generally expands at the location of weakness. The expansion further effects flow in the vicinity of the expansion. Upon further weakening of the vessel, the vessel can rupture due to the pressure in the vessel with corresponding deleterious effects. In some embodiments described herein, the blood vessel can be sculptured to more closely resemble the natural shape of the vessel along with stabilizing the tissue such that more normal function of the vessel can be expected.

The devices disclosed herein can be directed to localized delivery of therapeutic compositions to the stabilization of the elastin component of connective tissue, and in particular, blood vessels or other vessels. It should be understood that while a device can be directed in some embodiments to the stabilization of blood vessels susceptible to the formation of aneurysms, in other embodiments, other organs, other diseases and/or other conditions can be treated. In particular, the disclosed treatment agents and treatment protocols may be applicable to any animal or human connective tissue that includes an elastin component of a vessel.

As described herein, less invasive procedures can be used to deliver chemical stabilizing agents to stabilize the tissue in the vicinity of the aneurysm. Some level of structural remolding can be performed in conjunction with the chemical stabilization. In contrast, surgical treatment of aneurysms can involve endovascular stent graft repair (placement of a tube inside the vessel) or complete replacement of the diseased aorta or other blood vessel with an artificial vascular graft. Surgical treatment of aneurysms saves thousands of lives every year and improves quality of life. However, survival rates can drop to only 50% at 10 years post-operative due to surgery-related complications or device-related problems. In addition, endovascular stents are anatomically appropriate for only 30% to 60% of AAA patients at the outset and present the risk of endoleaks and graft displacement. Moreover, open surgery for full-size graft insertion is highly invasive, limiting its use to those patients with the ability to tolerate high operative risk. Early interventions for these potentially debilitating and life-threatening vascular pathologies may be advantageous since age is one of the major risk factors associated with the current approaches to treat aneurysms.

Procedures for chemical stabilization treatment of aneurysms is described in U.S. Patent 7,252,834 to Vyavahare et al. (the '834 patent), entitled "Elastin Stabilization of Connective Tissue". The devices and exemplary methods herein provide in some embodiments for effective delivery of the compositions of the '834 patent as well as other stabilization agents and/or other treatment agents for blood vessel tissue. The devices described herein provide for the isolation of a section of blood vessel wall, such as in the vicinity of the aneurysm using a device that can be positioned at the treatment site using less invasive procedures through the vasculature. Thus, the proximal end of the device remains outside of the patient while the distal end of the device is inserted through the patient's vasculature to the treatment location.

The isolation of the blood vessel wall can be performed with device designs that provide for continued blood flow past the isolated region based on a specific conduit or an appropriately designed opening through the structure providing the isolated treatment location. Furthermore, the devices can be adapted to use for the treatment of diseases other than aneurysm. In contrast with the devices described herein, a device that can deliver a treatment fluid to a selected region of a vessel without isolating the region is described in published U.S. patent application 2007/0293937A to Biggs et al., entitled "Endoluminal Medical Device for Local Delivery of Cathepsin Inhibitors, Method of Making and Treating". However, if treatment fluid is released within the vessel without isolating the selected portion of the blood vessel, the treatment fluid is released into the bloodstream downstream from the selected region of the vessel for a significant systemic delivery of the treatment fluid.

The device generally comprises a shaft and a sealing element. The sealing element comprises an extendable portion and fluid exchange portion. The sealing portion generally has a low profile delivery configuration and a deployed, extended configuration that forms the isolated volume. Specifically, the deployed device contacts the vessel wall upstream and downstream from the selected region in the blood vessel to form the isolated volume. The fluid exchange portion provides for the removal and/or delivery of fluid from the isolated volume. The sealing element can form a flow by-pass such that fluid within the vessel can flow past the isolated volume when the device is deployed.

The isolation of the region of the blood vessel in the vicinity of the aneurysm can be achieved using an extendable structure or structures, such as balloons or the like, or using self-extending structures that achieve a desired configuration upon release within the vessel. Balloon-based devices generally include a lumen to deliver a liquid to inflate one or more balloons. The same lumen may or may not be used to deliver the therapeutic agent. A separate lumen can be used to aspirate blood from the isolated region of the vessel. In some embodiments, the balloon is inflated with an inert liquid through a first lumen, and aspiration and therapeutic liquid delivery is performed through a second lumen. If aspiration and fluid delivery are performed sequentially through a single lumen, the lumen can be collapsible with aspiration to remove liquid from the lumen such that the treatment liquid can be delivered to the isolated volume. Alternatively or additionally, one or more micro-catheters can be delivered through a lumen to access the isolated volume with the micro-catheters such that the micro-catheters can be used to aspirate the isolated volume or to deliver a therapeutic agent.

In some embodiments, the device comprises one or more self-extending elements operably connected to a suitable membrane. The self-extending elements can be released from a sheath or using an actuation tool. For example, a spring metal frame can resume an extended configuration upon release from the sheath. Similarly, a tool can be used to transition a frame from a delivery configuration to a deployed configuration. The self-extending elements generally extend to the walls of the vessel to form a volume enclosed with the membrane and the self-extending element with a section of vessel wall forming a portion of the boundary of the enclosed volume.

The section of vessel wall associated with the enclosed volume generally includes a selected portion of the vessel wall for treatment, such as at least a portion of the aneurysm. The fluid exchange portion of the device provides for access to the isolated volume. One or two lumen associated with the shaft of the device extending from at or near the proximal end of the device provide for fluid communication to the fluid exchange portion and correspondingly into the enclosed volume. Specifically, a lumen can be used to withdraw blood from the region, and the same lumen or a separate lumen can be used to deliver a therapeutic agent.

The aneurysm generally is first identified using appropriate imaging techniques. The device can be introduced into the vessel using techniques for the delivery of catheters and the like using less invasive procedures. The sealing portion of the device is oriented within the vessel to be positioned in the vicinity of the aneurysm so that the resulting enclosed volume encompasses at least a portion of the aneurysm. In some embodiments, blood can be withdrawn from the enclosed volume. Withdrawal of the blood from the enclosed volume reduced the pressure below the blood pressure within the vessel. If the vessel has appropriate elasticity, the aneurysm can respond to the reduced pressure by reducing the volume of the aneurysm with at least partial return of the vessel to its natural shape. Also, a therapeutic agent can be introduced into the enclosed volume. The therapeutic agent can stabilize the tissue from further degradation and mechanical instability. The tissue stabilization can reduce the chance of rupture of the aneurysm with corresponding serious or potentially fatal consequences. Through the use of the combination of withdrawal of blood and the addition of a stabilization agent, the aneurysm can be stabilized in a less distorted shape.

The devices and corresponding processes described herein can provide treatments to inhibit and/or reverse the progression of aneurysm, prevent further weakening and dilation of the vessel wall. These procedures can be carried out in a less invasive format that reduces the recovery time and risk of the procedure to the patient.

### Device for the Delivery of Elastin Stabilization Agent

The delivery devices described herein provide for the introduction into a blood vessel using less invasive procedures. The distal end of the device can be positioned near an aneurysm or other location for treatment within a vessel. In general, the device comprises a shaft, a sealing element and flow lumen(s) that provide flow passages between locations at or near the distal end to locations at or near the proximal end. The one or more flow lumens extend through the shaft to provide for delivery to and/or removal of fluids from the isolated volume of the vessel. The sealing element is configured to isolate a volume against the wall of the vessel at a selected location such that the aneurysm or other region of interest can be accessed for localized delivery of a treatment fluid. Generally, the device further comprises a passageway that provides for vessel flow past the sealing device. The device optionally can comprise a guide lumen for the over-the-wire or rapid exchange interface of the device with a guidewire. While the device can be effective for the treatment of an aneurysm, the device can be used in other circumstances for the localized treatment of a portion of a blood vessel.

The shaft can have appropriate dimensions for delivery into the patient's blood vessels. In particular, the diameter of the shaft should be small enough to pass reasonably into desired vessels. The length of the shaft can be selected to reach desired locations while maintaining an appropriate portion of the device extending from the patient. The shaft can have a selected number of flow lumens to provide for the delivery and/or removal of fluids from near the distal end based on manipulations at the proximal end extending from the patient. For embodiments of particular interest, at least one lumen is needed for the delivery of the treatment compositions to the isolated volume either directly or using a micro-catheter.

In general, the shaft can have 1, 2, 3 or more distinct flow lumens. If three flow lumens are used, one flow lumen can be used to extend a balloon or the like to extend the sealing element, a second flow lumen can be used to aspirate blood from the isolated volume and a third flow lumen can be used to deliver the therapeutic composition. If the device has a self extending sealing element, rather than a balloon type structure, then the lumen that is used to provide fluid to extend a balloon or the like is not needed. Furthermore, the function of lumens can be combined in some embodiments. For example, a single lumen can be used to extend one or more balloons of a sealing structure, and the same lumen can be used to simultaneously deliver a therapeutic composition through slowly leaking the fluid into the isolated volume while maintaining an appropriate pressure in the balloon(s). Also, the isolated volume can be aspirated using a lumen that is also used to deliver the therapeutic composition. If a single lumen is used to both aspirate and to deliver a fluid, the flow lumen can be formed from a material that collapses under sufficient aspiration such that the therapeutic fluid can be delivered without being blocked by a lumen filled with blood or the other bodily fluid. If a single lumen is used for multiple functions then the number of lumen can be correspondingly reduced. The multiple functionality of a single lumen can be provided with a micro-catheter delivered to access the isolated volume in which the same or different micro-catheters can be used to aspirate and deliver therapeutic agents. In some embodiments, the micro-catheter can be pre-loaded with therapeutic agent before being delivered through the lumen to access the isolated volume.

Furthermore, the shaft can have a guide lumen for tracking the device over a guidewire or similar guide structure. In some embodiments, the device has a rapid exchange configuration such that the guide lumen only extends through a shorter section of the device near its distal end. The by-pass passageway that provides for flow through the vessel past the sealing element can also be used as a guide lumen. However, the device can have an over the wire configuration in which the guidewire extends through all or most of the length of the shaft.

The sealing element generally has an un-extended delivery configuration and an extended deployed configuration. In the extended deployed configuration, the sealing element isolates a volume within a portion of the vessel wall forming a boundary for the isolated volume. The isolated volume provides for the performance of treatment on an aneurysm without exposing the treatment compositions systemically. Furthermore, the isolated volume allows the localized decrease of pressure at the aneurysm. The sealing element can comprise two spaced apart components to seal independently two positions of the vessel. Alternatively, the sealing element can have two spaced apart components that have a sheet connecting them such that the end components contact the vessel walls and the sheet isolates the volume between the end components with the vessel wall forming an outer boundary of the isolated volume.

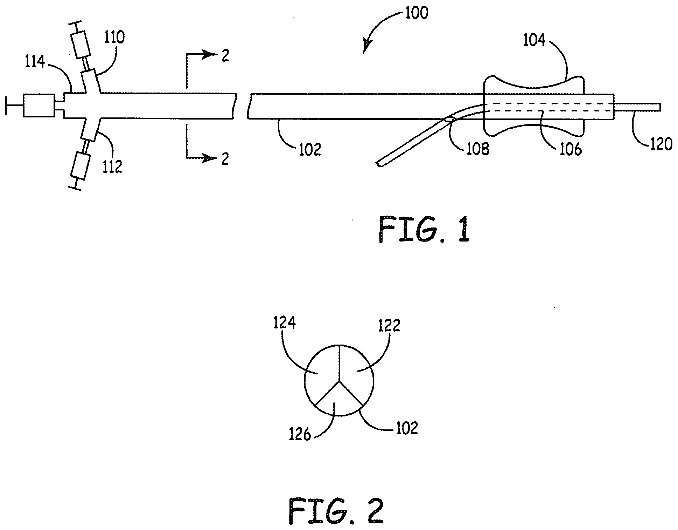

Referring to Fig. 1, a rapid exchange delivery device is shown schematically. Isolation/delivery device 100 comprises a shaft 102, a sealing element 104, a guide lumen 106 with a guide port 108, and three access ports 110, 112, 114 that provide for delivery or removal of fluids through three corresponding lumens. A guidewire 120 is shown extending through a separate guide lumen 106, which is attached to the shaft. Referring to Fig. 2, shaft 102 comprises three flow lumens 122, 124, 126 that, respectively, are in fluid communication with access ports 110, 112, 114. Referring to Fig. 1, access ports 110, 112, 114 are respectively connected to flow devices 116, 118, 120. The flow devices can be syringes, pumps, or the like, or combinations thereof. For example, an empty syringe can be used to withdraw fluid and a syringe with a liquid can be used to deliver the liquid to the isolated volume in the vessel. Luer fitting and other appropriate fittings, such as those known in the art, can be used to attach the flow devices to the access ports.

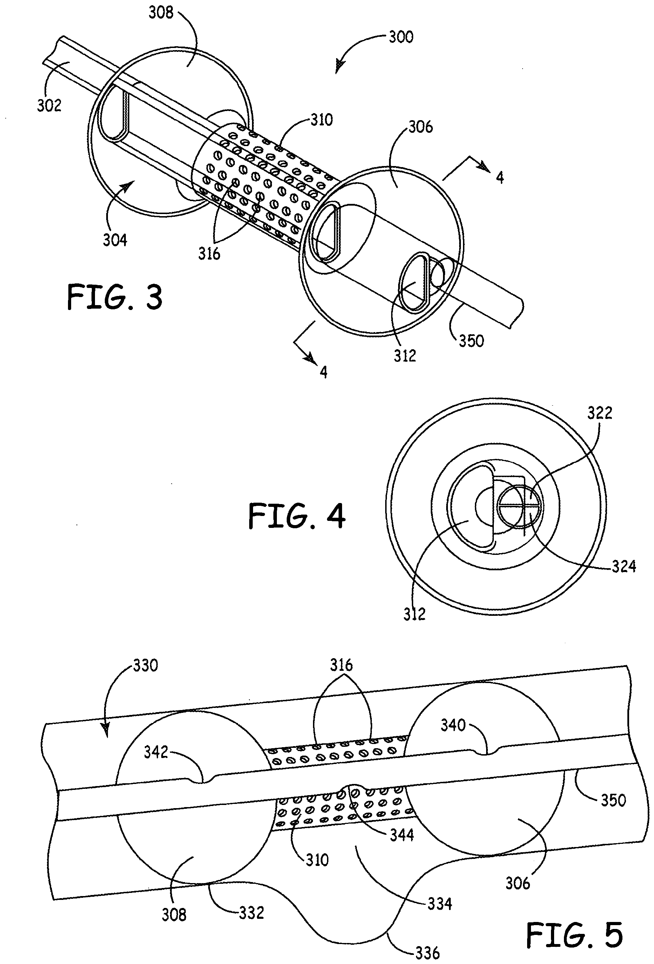

Referring to Fig. 3, the extended configuration of an exemplary embodiment of an isolation/delivery device is shown. Isolation/delivery device 300 comprises a shaft 302 and an extendable element 304 attached to the shaft. As shown in Fig. 4, the shaft comprises two lumens 322 and 324 that are in fluid communication with the extendable element 304. At or near the proximal end of the shaft, the lumens are in fluid communication, respectively, with separate ports that are connect to liquid delivery and/or suction devices.

Extendable element 304 comprises a distal balloon 306, a proximal balloon 308, a fluid exchange portion 310, and a by-pass channel 312. Lumen 322 is in fluid communication with balloons 306, 308 so that the inflation and deflation of the balloons can be controlled with fluid flowed through lumen 322. The fluid exchange portion 310 has a plurality of openings 316 that are in fluid communication with lumen 324. When deployed in a vessel, balloons 306, 308 form an isolated volume, and fluid flow into and out from the isolated volume can be controlled through fluid exchange portion 310.

Referring to Fig. 5, the device is extended by inflating extendable element 304 with fluid from lumen 322 in a vessel 330. In the extended configuration, the distal balloon 306 and the proximal balloon 308 push against vessel wall 332 to isolate a portion of the vessel to form an isolated volume 334 in the vicinity of aneurysm 336. Lumen 322 accesses distal balloon 306 through port 340 and proximal balloon 308 through port 342. Lumen 324 accesses fluid exchange portion 310 through port 344.

As shown in Fig. 3, by pass channel 312 allows vessel fluid such as blood to past through the extendable element 304. The plurality of openings 316 in the fluid exchange portion are in fluid communication with the isolated volume 334. The fluid in the isolated volume 334 can be aspirated through the openings 316 into lumen 324 to reduce the pressure in the isolated volume and potentially to shrink the expanded volume of aneurysm 336. After the aspiration, therapeutic composition can be delivered through lumen 324 to fluid exchange portion 310 to be released into the isolated volume 334 through the openings 316. In some embodiments, openings 316 of fluid exchange portion 310 can be replaced or supplemented with alternative liquid permeable structures. A flexible structure 350, which can be similar to a guidewire structure, can extend form the distal end of the device to facilitate delivery of the device, although in alternative or additional embodiments, the device can ride over a guidewire in an over the wire or a rapid exchange configuration.

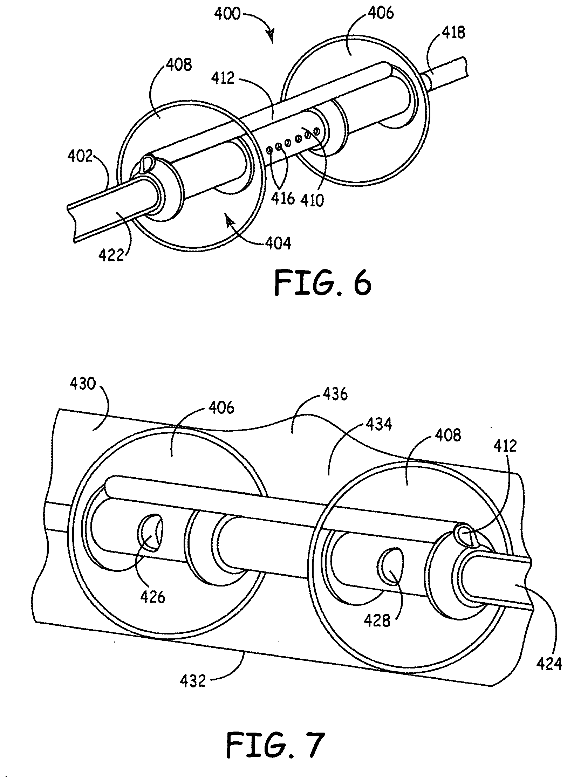

Referring to Fig. 6, the extended configuration of an exemplary embodiment of an isolation/delivery device is shown. Isolation/delivery device 400 comprises a shaft 402 and an extendable element 404. The shaft comprises two lumens 422, 424 that are in fluid communication with the extendable element 404. The device has an optional flexible guide wire like structure 418 to provide for directing into a vessel. At or near the proximal end of the shaft, the lumens of shaft 402 are in fluid communication with ports that are connectable, respectively to liquid delivery/removal devices.

Extendable element 404 comprises a distal balloon 406, a proximal balloon 408, a fluid exchange portion 410, and a by-pass channel 412. Lumen 424 is in fluid communication with balloons 406, 408 through ports 426, 428 so that the inflation and deflation of the balloons can be controlled with fluid flowed through lumen 424. The fluid exchange portion 410 has a plurality of openings 416 that are in fluid communication with lumen 422. In some embodiments, openings 416 of fluid exchange portion 410 can be replaced or supplemented with alternative liquid permeable structures. When deployed in a vessel, balloons 406, 408 form an isolated volume inside the vessel, and fluid flow into the isolated volume can be controlled through fluid exchange portion 410.

Referring to Fig. 7, the device is extended by inflating extendable element 404 with fluid from lumen 424 in a vessel 430. In the extended configuration, the distal balloon 406 and the proximal balloon 408 push against vessel wall 432 to isolate a portion of the vessel to form an isolated volume 434 in the vicinity of aneurysm 436. By pass channel 412 allows vessel fluid such as blood to past through the extendable element 404. The plurality of openings 416 (Fig. 6) in the fluid exchange portion are in fluid communication with the isolated volume 434. Therapeutic composition can be delivered through lumen 422 to be released into the isolated volume 434 through the openings 416.

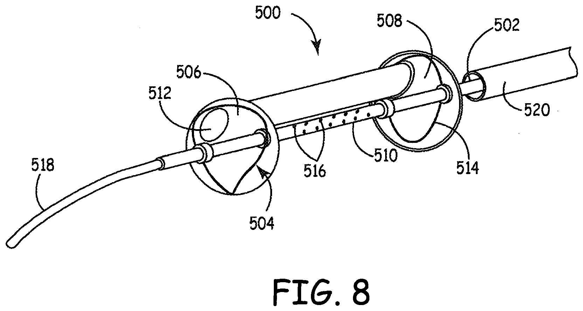

Referring to Fig. 8, the extended configuration of an exemplary embodiment of an isolation/delivery device is shown. Isolation/delivery device 500 comprises a shaft 502 and a self extending element 504 attached to the shaft. The device has an optional flexible guide wire like structure 518 to provide for directing into a vessel. The shaft comprises a fluid exchange portion 510 and a central lumen that is in fluid communication with liquid delivery and/or suction devices at or near the proximal end of the shaft.

Self extending element 504 comprises a distal extendable element 506, a proximal extendable element 508, and a by-pass channel 512. Extending and collapsing of the self extending element 504 is controlled through a catheter 520. In the collapsed configuration, self extending element 504 along with the shaft is tucked inside the catheter 520. Once the catheter 520 is retracted, the shape memory wires 514 extend to deploy self extending element 504. Fluid exchange portion 510 has a plurality of openings 516 that are in fluid communication with the lumen. When deployed in a vessel, self-extending elements 506, 508 push against vessel wall to isolate a portion of the vessel to form an isolated volume, and fluid flow into and out from the isolated volume can be controlled through fluid exchange portion 510. In addition, by pass channel 512 allows vessel fluid such as blood to flow past self extending element 504. When the device is deployed in the vicinity of an aneurysm, the fluid in the isolated volume can be aspirated through openings 516 into the lumen to reduce the pressure in the isolated volume and potentially to shrink the expanded volume of the aneurysm. After aspiration, therapeutic composition can be delivered through the lumen to fluid exchange portion 510 to be released into the isolated volume through the openings 516. In some embodiments, openings 516 of fluid exchange portion 510 can be replaced or supplemented with alternative liquid permeable structures.

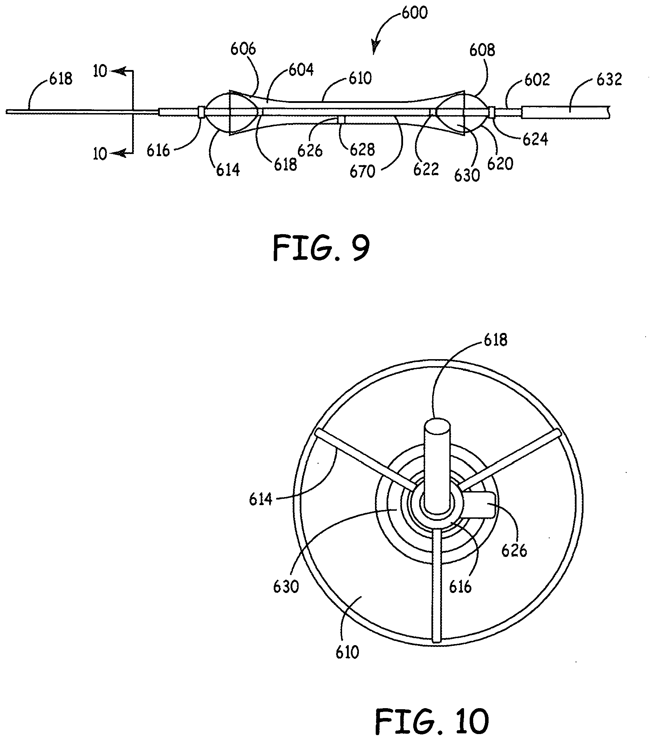

Referring to Figs. 9 and 10, the extended configuration of an exemplary embodiment of an isolation/delivery device 600 is shown. Isolation/delivery device 600 comprises a shaft 602 and a self extending element 604 attached to the shaft. The device has an optional flexible guide wire like structure 618 to provide for directing into a vessel. Shaft 602 comprises a fluid exchange portion 670 and a central lumen that is in fluid communication with liquid delivery and/or suction device(s) at or near the proximal end of the shaft.

Self extending element 604 comprises a distal frame 606, a proximal frame 608 and a fluid impermeable membrane 610. Membrane 610 can be formed from a non-porous polymer sheet, a metal foil, combinations thereof or the like. Distal frame 606 comprises shape memory wires 614 attached to shaft 602 at anchors 616, 618, and proximal frame 608 comprises shape memory wires 620 attached to shaft 602 at anchors 622, 624. The central lumen of shaft 602 opens to the exterior of membrane 610 through tube 626 that is in fluid communication with the central lumen and has an opening 628 at its tip. The space between shaft 602 and membrane 610 forms a flow by-pass channel 630 for flow in a vessel to pass self extending element 604. Extending and collapsing of the self extending element 604 is controlled with a catheter 632. In the collapsed configuration, self extending element 604 is collapsed inside catheter 632 with wire frames 614, 620 in a low profile configuration constrained by the catheter. Once catheter 632 is retracted, shape memory wires 614, 620 extend outward to deploy the extendable element 604 with contact against the vessel wall to form an isolated volume between the vessel wall and the membrane.

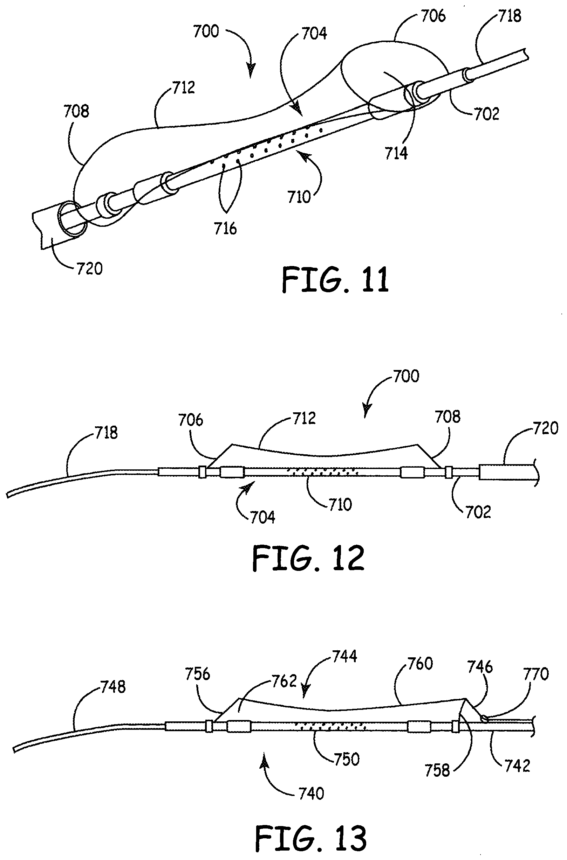

Referring to Figs. 11 and 12, the extended configuration of another exemplary embodiment of an isolation/delivery device are shown. Isolation/delivery device 700 comprises a shaft 702 and a self extending element 704 attached to the shaft. The device has an optional flexible guide wire like structure 718 extending from the distal end of the shaft to provide for directing the device into a vessel. The shaft comprises a fluid exchange portion 710 and a central lumen that is in fluid communication with liquid delivery and/or suction device(s) at or near the proximal end of the shaft.

Self extending element 704 comprises a distal support 706, a proximal support 708, and a nonporous membrane 712. Nonporous membrane 712 forms an isolated volume around a section of the vessel wall upon deployment. Extending and collapsing of the self extending element 704 is controlled with a catheter 720. In the collapsed configuration, self extending element 704 is collapsed inside the catheter 720. Once the catheter 720 is retracted, self extending element 704 extends to form a by pass flow channel 714. Fluid exchange portion 710 has a plurality of openings 716 that are in fluid communication with the lumen extending through the shaft. When deployed in a vessel, supports 706, 708 extend against vessel wall to isolate a portion of the vessel to form an isolated volume, and fluid flow into and out from the isolated volume can be controlled through fluid exchange portion 710. Furthermore, by pass channel 714 allows vessel fluid such as blood to past through the extended element 704. When the device is deployed in the vicinity of an aneurysm, the fluid in the isolated volume can be aspirated through openings 716 into the lumen to reduce the pressure in the isolated volume and potentially to shrink the extended volume of the aneurysm. After aspiration, therapeutic composition can be delivered through the lumen to fluid exchange portion 710 to be released into the isolated volume through the openings 716. In some embodiments, openings 716 of fluid exchange portion 710 can be replaced or supplemented with alternative liquid permeable structures.

Fig. 13 shows an exemplary embodiment similar to Figs. 11 and 12 although with an actuation element. Referring to Fig. 13, isolation/delivery device 740 comprises a shaft 742, an extending element 744 attached to the shaft and an actuation element 746. The device has an optional flexible guide wire like structure 748 extending from the distal end of the shaft to provide for directing the device into a vessel. The shaft comprises a fluid exchange portion 750 and a central lumen that is in fluid communication with liquid delivery and/or suction device(s) at or near the proximal end of the shaft.

Self extending element 744 comprises a distal support 756, a proximal support 758, a connecting support 760 connecting distal support 756 and proximal support 758, and a nonporous membrane 762. Nonporous membrane 762 forms an isolated volume around a section of the vessel wall upon deployment. Fluid exchange portion 750 has a plurality of openings that are in fluid communication with the lumen extending through the shaft.

Proximal support 758 is connected to actuation element 746, which can be a wire or the like. Actuation element 746 extends along shaft 742 with loops 770 guiding the actuation element, although loops 770 can be replaced with an actuation element lumen. If actuation element 746 is pulled in a proximal direction, proximal support 758 and distal support 756 collapse to a delivery configuration due to the elastic nature of the supports. Upon movement of the actuation element in a distal direction, the supports transition to a deployed configuration, as shown in Fig. 13.

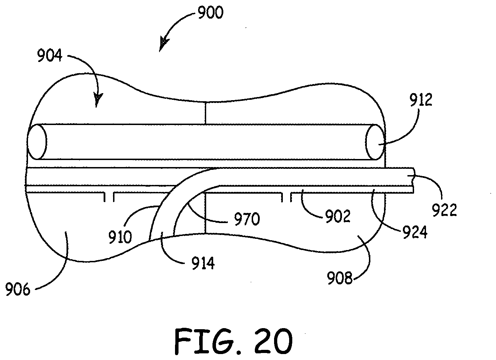

Referring to Fig. 20, the extended configuration of an embodiment of an isolation/delivery device 900, according to the invention, is shown. Isolation/delivery device 900 comprises a shaft 902 and an extendable element 904 attached to the shaft. The shaft comprises an access lumen 922, a balloon lumen 924 and a fluid exchange portion 970. The fluid exchange portion 970 comprises a distal side channel portion 910 connected to access lumen 922 having a side opening 914 on the side of the extendable element 904. In one embodiment, access lumen 922 ends at the fluid exchange portion 970 to form a smooth connection with the side channel 910. Extendable element 904 comprises a distal balloon 906, a proximal balloon 908, a bypass channel 912 and the side opening 914. Balloon lumen 924 is in fluid communication with balloons 906, 908 so that the inflation and deflation of the balloons can be controlled with fluid flowed through said lumen.

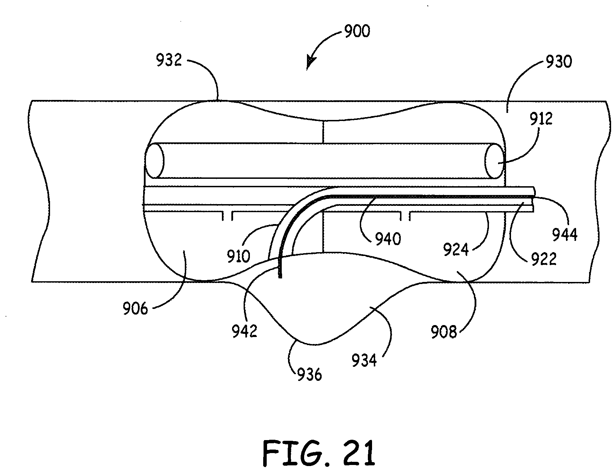

Referring to Fig. 21, the device of Fig. 20 is deployed in the vicinity of an aneurysm 936 inside a vessel 930. In the extended configuration, the distal balloon 906 and the proximal balloon 908 push against vessel wall 932 to isolate a portion of the vessel 930 to form an isolated volume 934 in the vicinity of the aneurysm 936. A micro-catheter 940 is placed inside access lumen 922 through side channel 910 and side opening 914 to reach isolated volume 934, comprising a distal end 942 and a proximal end 944.

In one embodiment, a first micro-catheter 940 is used to aspirate fluid from the isolated volume 934 through an aspiration apparatus attached to the proximal end 944 of the micro-catheter. The first micro-catheter 940 is then replaced with a second micro-catheter 940 to deliver a stabilizing agent into the isolated volume 934 through a delivery device attached to the proximal end 944 of the second micro-catheter. In one embodiment, the second micro-catheter 940 is pre-loaded with stabilizing agent. One or more of the steps can be repeated as described.

To provide for visualization of the device within the patient, the device or selected portions thereof can be formed from a radio opaque material that can be visualized using imaging techniques, such as x-ray imaging. Generally, it can be desirable to include specific imaging markers at or around the sealing element since the placement of the sealing element is directed to the isolation of a selected volume. Thus, marker bands, radio opaque components or the like can be placed at or in the vicinity of the sealing elements to assist with placement of the sealing elements at the desired location within a vessel of the body.

The delivery device can be formed from one or more biocompatible materials, including, for example, metals, such as stainless steel or alloys, e.g., Nitinol®, or polymers such as polyether-amide block co-polymer (PEBAX®), nylon (polyamides), polyolefins, polytetrafluoroethylene, polyesters, polyurethanes, polycarbonates or other suitable biocompatible polymers. Radio-opacity can be achieved with the addition of markers, such as platinum-iridium or platinum-tungsten or through radio-pacifiers, such as barium sulfate, bismuth trioxide, bismuth subcarbonate, powdered tungsten, powdered tantalum or the like, added to the polymer resin. Generally, different sections of the shaft can be formed from different materials from other sections, and sections of the shaft can comprise a plurality of materials at different locations and/or at a particular location. Balloons and the like can be formed from suitable elastic polymers and the like. Porous membranes for the formation of some embodiments of fluid exchange portions can be formed from polymer sheets or the like with selected holes placed within the sheet with the number and size of the holes placed to provide the desired degree of fluid passage.

### Procedure for Isolating a Volume and Providing Localized Treatment

A procedure of using the device described herein generally comprises the steps of introducing the device into a blood vessel, placing the device near the site of aneurysm, activate the device to isolate the site of aneurysm, aspirate blood from around the site of aneurysm, deliver an effective amount of a therapeutic agent into the site of the aneurysm, deactivate the device, and withdraw the device from the blood vessel. Optionally steps that aspirate blood from around the site of aneurysm and deliver an effective amount of a therapeutic agent into the site of the aneurysm can be repeated to achieve desired effects. The therapeutic composition delivered can be the same or different compositions. For example, the delivery of one therapeutic composition comprises the delivery of an elastin stabilization composition and the delivery of the other therapeutic composition comprises the delivery of glutaraldehyde, which can be sequentially delivered in a desired order. To identify the location for placement of the device, appropriate imaging is performed prior to performing the procedure as well as during the procedure.

While the device can be used for other procedures, the discussion below focuses on the treatment of an aneurysm since the treatment of aneurysms is an issue of very significant clinical concern, and useful treatment agents have recently been developed. However, other vessel diseases or damage can be treated through the formation of an isolated volume. For example, a calcified portion of a vessel can be treated through the delivery of a thrombolytic agent, such as tissue plasminogen activator (tPA) or urokinase, or a mild acid or anti-calcification enzymes such as osteopontin to resorb calcific plaque.

With respect to aneurysms, recent techniques have been developed to track the progress of the aneurysm using a blood test. This is described further in U.S. patent application publication number 2009/0186370 A1 to Ogle et al., entitled "Diagnostic Biomarkers for Vascular Aneurysm". Once the aneurysm is identified and has progressed to a stage of initiating treatment, imaging generally is used to identify the location of the aneurysm and to assess the severity of the problem and to identify an approach for the treatment procedure.

Methods for diagnosing and identifying the degree of aneurysm expansion are available due to developments in high resolution imaging technology (CT, MRI). Various appropriate contrast agents can be used to enhance the imaging. The use of magnetic resonance and CT imaging techniques to guide procedures on aneurysms is described further in U.S. Patent 6,463,317 to Kucharczyk et al., entitled "Device and Method for the Endovascular Treatment of Aneurysms," and U.S. Patent 6,793,664 to Mazzocchi et al., entitled "System and Method of Minimally-Invasive Exovascular Aneurysm Treatment".

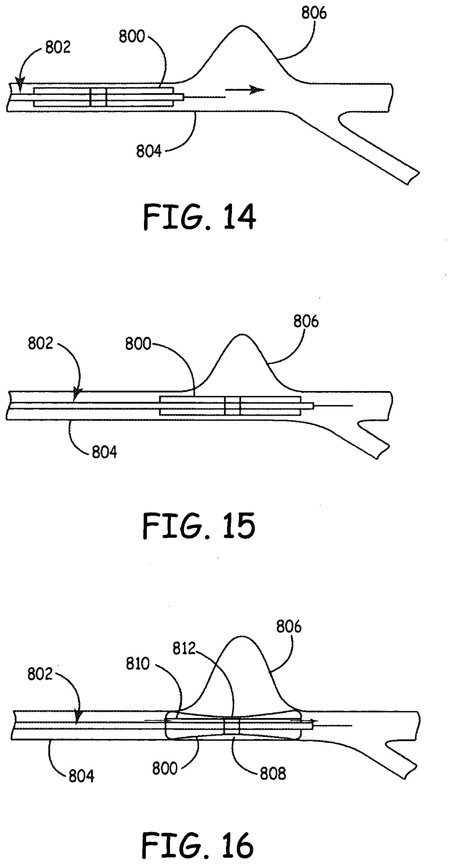

Based on the identified location of the aneurysm, the procedure can be performed to direct the device to the aneurysm for the delivery of a stabilization agent. Referring to Fig. 14, exemplary sealing element 800 of delivery device 802 is delivered into vessel 804 with an aneurysm 806. Sealing element 800 is shown in a low profile delivery configuration. Referring to Fig. 15, exemplary sealing element 800 is placed in the vicinity of aneurysm 806. Radio opaque markers on device 800 can be used to perform imaging during the procedure to locate the position of sealing element 800 within the vessel.

Referring to Fig 16, sealing exemplary element 800 is shown in an extended configuration forming an isolated volume 808. The transition to the extended configuration can be performed based on the particular design of the device. For example, the transition to the extended configuration can be performed, for example, through the filing of one or more balloons, through the release of a self extending member from a sheath or through the use of an actuation element.

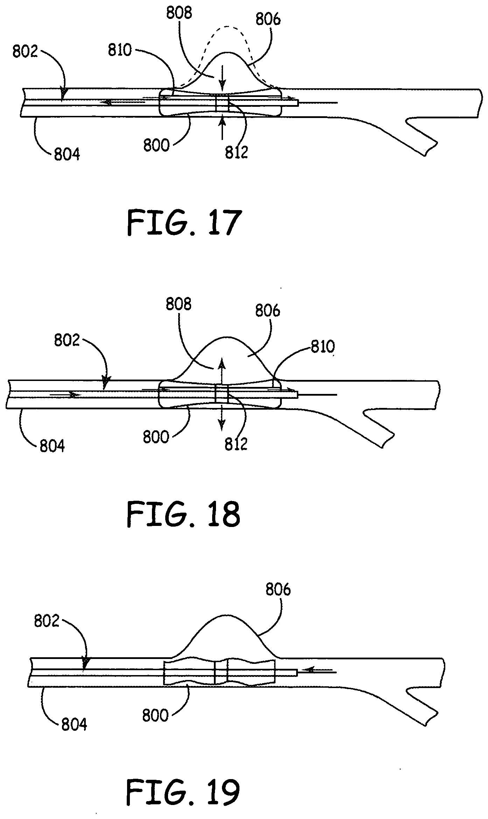

Flow in the vessel is maintained through a by-pass channel 810. Fluid exchange portion 812 is configured for the exchange of fluids between a lumen of device 802 and isolated volume 808. In an optional step shown in Fig. 17, blood is withdrawn from isolated volume 808 through the fluid exchange portion 812 and a lumen in device 802. The withdrawal of blood decreases the pressure in isolated volume 808, which can result in decrease or elimination of the distortion of the vessel at the aneurysm 806.

Referring to Fig. 18, a stabilization composition is delivered into isolated volume 808 where it can interact with aneurysm 806 to stabilize the vessel at the aneurysm. The stabilization composition is delivered through a lumen in device 802 through fluid exchange portion 812. Since flow is maintained in the vessel through by-pass channel 810, a reasonable period of time can be provided for the stabilization composition to act on the vessel wall. In some embodiments, the treatment can last for a period of time from about 1 minute to about 5 hours, in further embodiments from about 5 min. to about 60 min., and in additional embodiments from about 15 min. to about 30 min. A person of ordinary skill in the art will recognize that additional ranges of treatment times within the explicit ranges above are contemplated and are within the present disclosure. With respect to embodiments in which the aspirating step and/or the delivering of the therapeutic composition steps are repeated at least once and in which the therapeutic compositions delivered can be the same or different compositions, the treatment times discussed herein for the repeated steps can be the same or different. For example, the delivery of one therapeutic composition can comprise the delivery of glutaraldehyde which is allowed to act on the vessel for example, from about 5 min to about 30 min and the delivery of the other therapeutic composition comprises the delivery of an elastin stabilization composition which is allowed to act on the vessel for example from about 15 min. to about 30 min.

Referring to Fig. 19, once the selected period of time has passed for providing contact with the stabilization composition, the isolated volume 808 can be optionally aspirated, using similar process as described in Fig. 17. The exemplary sealing element can be transitioned to a recovery configuration, which can approximate the delivery configuration, with a lower profile and without forming an isolated volume. The transition to the recovery configuration can comprise, for example, the deflation of one or more balloons, the folding of a compliant frame using a sheath or the like, or the use of an actuating member to transition the element. Once the sealing element is transitioned to the recovery configuration, device 802 can be removed form the patient.

In general, the exemplary procedure outlined in Figs. 14-19 can be performed with any of the exemplary devices shown in Figs. 3-13 as well as alternative embodiments discussed herein. The procedures generally vary in minor respects based on the nature of the sealing element. In some embodiments, additional steps of delivering and removing liquids from the isolated region can be performed if desired, such as for the sequential contact with stabilization fluids. In one embodiment, the step of delivering the therapeutic composition is repeated with a different composition, wherein the delivery of one therapeutic composition comprises the delivery of an elastin stabilization composition and the delivery of the other therapeutic composition comprises the delivery of glutaraldehyde.

### Formulation and Delivery Options

Unless otherwise noted, all concentrations reported herein are weight/volume percentages in which the weights are in grams and the volumes are in milliliters. For example, 0.06% pentagalloylglucose (PGG) means 0.06 g PGG in 100 mL of water or 100 mL of saline or other liquid carrier. In general, suitable elastin stabilizing therapeutic compositions can be provided in pharmaceutically acceptable formulations, such as using formulation methods known to those of ordinary skill in the art. These formulations can generally be administered to connective tissue associated with an isolated volume in the vicinity of an aneurysm through the device described herein. The therapeutic compositions can comprise a phenolic compound that interacts with elastin to stabilize the tissue.

Once delivered to the targeted blood vessel by any suitable exemplary method, the composition can access and then stabilize the connective tissue of the vessel. For instance, when delivered to the connective tissue from the lumen of a blood vessel, the composition disclosed herein may penetrate the endothelium of the vessel wall to contact the elastin of the connective tissue and stabilize the structure architecture. While the therapeutic compositions can be delivered using the devices described herein, in alternative or additional embodiments, the compositions can be delivered intravenously in a systemic delivery protocol. For example, osmotic mini-pumps may be used to provide controlled delivery of high concentrations of the treatment compositions through cannulae to the site of interest, such as directly into a targeted blood vessel. Regardless of the delivery approach, *in situ* polymerizable hydrogels, as are generally known to those of skill in the art, and discussed further below, are another example of a delivery vehicle that can be utilized in a delivery protocol.

Therapeutic compositions can include additional agents, in addition to agents that stabilize elastin. Such additional agents can be active agents, providing direct benefit to the tissue, or may be supporting agents, improving delivery, compatibility, or reactivity of other agents in the composition. For example, in one embodiment, the compositions can include glutaraldehyde. Glutaraldehyde, when targeted to connective tissue, can form covalent crosslinks between free amines in proteins in order to further stabilize the tissue. In another embodiment, glutaraldehyde can be delivered before or after the tissue being treated with an elastin stabilization composition. If desired, the composition can incorporate a gallic acid scavenger, for example ascorbic acid or glutathione, so as to decrease or prevent the release of free gallic acid residues. Also, the therapeutic composition can be combined with any of a number of possible lipid-lowering medications so as to prevent the development of calcified lipid deposits or arteriosclerosis plaques that can often be found in conjunction with aneurysm formation.

The additional agents can have a concentration from about 0.0001% to about 10%. It should be noted, however, that while these exemplary concentrations are effective in certain embodiments, compositions used herein comprises a wider range of concentrations. For example, actual concentrations used may be influenced by the organ targeted by the procedure, size of the targeted area, desired incubation time, and preferred pH, in addition to delivery mode, as mentioned above. In one embodiment, the disclosed compositions can comprise concentrations of the additional agent ranging from about 0.01% to about 2% and in additional embodiments from about 0.1% to about 1 %. A person of ordinary skill in the art will recognize that additional ranges within the explicit ranges above are contemplated and are within the present disclosure.

The therapeutic composition can comprise one or more buffers. For example, a composition comprising one or more phenolic compounds as an elastin stabilizing agent and having a pH from about 4.0 to about 9.0 may be formulated with inclusion of purified water, saline and a biocompatible buffer, such as phosphate buffers, borate buffers, HEPES, PIPES, and MOPSO. In one embodiment, a composition may be formulated to have a pH of between about 5.5 and about 7.4.

Compositions for parenteral delivery, e.g., via injection or localized delivery in the vasculature with the devices herein, can include pharmaceutically acceptable sterile aqueous or nonaqueous solutions, dispersions, suspensions or emulsions as well as sterile powders for reconstitution into sterile injectable solutions or dispersions just prior to use. Examples of suitable aqueous and nonaqueous carriers, diluents, solvents or vehicles include water, ethanol, polyols (e.g., glycerol, propylene glycol, polyethylene glycol and the like), carboxymethylcellulose and suitable mixtures thereof, vegetable oils (e.g., olive oil) and injectable organic esters such as ethyl oleate. In addition, the composition can contain minor amounts of auxiliary substances such as wetting or emulsifying agents, pH buffering agents and the like that can enhance the effectiveness of the phenolic compound. Proper fluidity may be maintained, for example, by the use of coating materials such as lecithin, by the maintenance of the required particle size in the case of dispersions and by the use of surfactants. These compositions may also contain adjuvants such as preservatives, wetting agents, emulsifying agents and dispersing agents.

Prevention of the action of microorganisms may be ensured by the inclusion of various antibacterial and antifungal agents such as paraben, chlorobutanol, phenol, sorbic acid and the like. It may also be desirable to include isotonic agents such as sugars, sodium chloride and the like.

In some embodiments, the compositions can include pharmaceutically acceptable salts of the components therein, e.g., those that may be derived from inorganic or organic acids. Pharmaceutically acceptable salts are well known in the art. For example, S. M. Berge, et al. describes pharmaceutically acceptable salts in detail in J. Pharmaceutical Sciences (1977) 66:1 et seq. Pharmaceutically acceptable salts include the acid addition salts that are formed with inorganic acids such as, for example, hydrochloric or phosphoric acids, or such organic acids as acetic, tartaric, mandelic and the like. Salts formed with free carboxyl groups can also be derived from inorganic bases such as, for example, sodium, potassium, ammonium, calcium or ferric hydroxides, and such organic bases as isopropylamine, trimethylamine, 2-ethylamino ethanol, histidine, procaine and the like. Representative acid addition salts include, but are not limited to acetate, adipate, alginate, chloride, sulfate, citrate, aspartate, benzoate, benzenesulfonate, bisulfate, butyrate, camphorate, camphorsulfonate, digluconate, glycerophosphate, hemisulfate, heptonoate, hexanoate, fumarate, hydrochloride, hydrobromide, hydroiodide, 2-hydroxymethanesulfonate (isethionate), lactate, maleate, methanesulfonate, nicotinate, 2-naphthalenesulfonate, oxalate, pamoate, pectinate, persulfate, 3-phenylpropionate, picrate, pivalate, propionate, succinate, tartate, thiocyanate, phosphate, glutamate, bicarbonate, p-toluenesulfonate and undecanoate. Also, the basic nitrogen-containing groups can be quaternized with such agents as lower alkyl halides such as methyl, ethyl, propyl, and butyl chlorides, bromides and iodides; dialkyl sulfates like dimethyl, diethyl, dibutyl, and diamyl sulfates; long chain halides such as decyl, lauryl, myristyl and stearyl chlorides, bromides and iodides; arylalkyl halides like benzyl and phenethyl bromides and others.

### Elastin Stabilization Agents

Elastin is a protein constituent of connective tissue contributing to the elasticity and recoil of the tissue. Moreover, elastin is quite abundant in connective tissue. Elastin is considered the most abundant extracellular matrix protein found in the aortic wall. Elastin polypeptide chains are naturally cross-linked together to form rubber-like, elastic fibers. Unlike collagen, elastin molecules can uncoil into a more extended conformation when the fiber is stretched and will recoil spontaneously as soon as the stretching force is relaxed. Elastin degeneration in connective tissue pathology is generally believed to be caused by enzymes including elastase enzymes and matrix metalloproteinase (MMP) enzymes that can be secreted by vascular cells as well as by infiltrating inflammatory cells. While many aspects of the methods and schemes of various enzymes leading to elastin degradation remain unknown, in general, it is believed that most enzymes attack and bind the protein at a site away from the natural crosslinks.

### Elastin Degeneration within Aneurysms

The characteristics of aneurysms are degeneration of arterial structural proteins including elastin and collagen, inflammatory infiltrates, calcification, and overall destruction of arterial architecture. This results in loss of mechanical properties and progressive dilatation. Due to its insolubility, natural desmosine and isodesmosine crosslinks, and extremely long biological half-life, elastin is generally perceived to be resistant to degradation. However, there are a specific set of enzymes, matrix metalloproteinases (particularly MMP-2, MMP-9, and MMP-12), which are capable of degrading elastin. MMPs are involved in normal physiological processes such as bone remodeling, wound healing, and angiogenesis. However, abnormally high levels of MMPs have been identified in pathological processes in many vascular diseases, and appear to be significant contributors to the formation and progression of AAAs. This fact is underlined by consistent reports of severe elastin degradation within these aneurysmal tissues, as evidenced by heavy degeneration of the arterial architecture, decreased medial elastin content, and disrupted or fragmented elastic lamellae. This degradation is particularly significant when one considers the inability of elastin to promptly revitalize itself (as evidenced by its nearly 70-year biological half-life), unlike some other relatively dynamic matrix components.

Furthermore, degradation of elastin results in the release of soluble elastin peptides. These peptides are not passive by-products of the degradation process; rather, it has been demonstrated that they are active in protease production, chemotaxis, cellular proliferation, and various other biological activities. The release of elastin peptides can result in a cascade of even more matrix degradation, as it has been shown that interactions between these peptides and smooth muscle cells increase expression of the elastin laminin receptor (ELR). This binding with ELR, a 67 kDa receptor found on a number of cell types, subsequently results in the promotion of greater MMP synthesis both at the mRNA and protein levels. Numerous studies have confirmed this correlation between upregulated MMP activity and the presence of elastin peptides. The use of luminally-perfused elastin peptides as an aneurysm animal model, which elicits elevated MMP levels and matrix degradation at the site of perfusion, also solidifies the biological power of these peptides. The extreme bioactivity of elastin peptides underscores the clinical significance of elastin degradation within aneurysmal tissues and the subsequent need to protect elastin from degeneration.

Degradation of connective tissue can be prevented or slowed through the stabilization of the elastin component of the tissue with a phenolic compound delivered by the device. This beneficial effect is described further in U.S. patent application publication number 2009/0214654 A1 to Isenburg et al., entitled "Treatment of Aneurysm With Application of Elastin Stabilizing Agent Embedded in a Delivery System". In particular, it is believed that any of a number of natural and synthetic phenolic compounds can bind elastin and thereby protect elastin from degradation, for instance due to the action of elastin degrading enzymes. Accordingly, in one embodiment, the present disclosure is directed to device and exemplary methods that deliver compositions that can inhibit enzyme-catalyzed degradation of elastin, and in particular elastase and/or MMP catalyzed degradation of elastin.

### Phenolic Compounds as Elastin Stabilization Agents

Phenolic compounds are a diverse group of materials that have been recognized for use in a wide variety of applications. For instance, they naturally occur in many plants, and are often a component of the human diet. Phenolic compounds have been examined in depth for efficacy as free radical scavengers and neutralizers, for instance in topical skin applications and in food supplements.

Phenolic compounds in some embodiments include any compound that includes at least one phenolic group bound to a hydrophobic core. While not wishing to be bound by any particular theory, it is believed that interaction between the phenolic compound and elastin proteins include aspects involving both the hydroxyl group as well as the hydrophobic core of the molecules. In particular, it is believed that phenolic compounds can stabilize elastin proteins through both steric means and bond formation and thereby protect sites on the protein susceptible to enzyme-mediated (e.g., elastase or MMP-mediated) cleavage. Specifically, it is believed that hydroxyl groups of a phenolic compound can bind elastin multivalently, for instance via hydrogen bond formation with amino acid residues such as polar amino acid residues including methionine, glycine and proline, such that multiple proteins can interact with a single molecule to create a three-dimensional cross-link structure involving multiple elastin molecules. Moreover, in certain embodiments, the phenolic compounds can include one or more double bonds, with which the phenolic compounds can covalently bind to the elastin, forming an even stronger and more permanent protective association between the phenolic compound and the elastin of the connective tissue.

In addition, the large hydrophobic regions of the elastin protein, which are believed to contain sites susceptible to elastase-mediated cleavage, are also believed to contain sites of association between the hydrophobic core of the phenolic compound and the protein. Thus, the association between the phenolic compound and the protein molecules are believed to protect specific binding sites on the protein targeted by enzymes through the association of the protein with the hydrophobic core and can also sterically hinder the degradation of the protein through the development of the large three dimensional cross-link structures.

Phenolic compounds encompassed herein include materials including a hydrophobic core and one or more phenol groups extending from the hydrophobic portion of the molecule. For instance, exemplary phenolic compounds can include, but are not limited to, flavonoids and their derivatives (e.g., anthocyanins, quercetin), flavolignans, phenolic rhizomes, flavan-3-ols including (+)-catechin and (-)-epicatechin, other tannins and derivatives thereof (such as tannic acid, pentagalloylglucose, nobotanin, epigallocatechin gallate, and gallotannins), ellagic acid, procyanidins, and the like.

Phenolic compounds encompassed herein also include synthetic and natural phenolic compounds. For example, natural phenolic compounds can include those found in extracts from natural plant-based sources such as extracts of olive oil (e.g., hydroxytyrosol (3,4-dihydroxyphenylethanol) and oleuropein, extracts of cocoa bean that can contain epicatechin and analogous compounds, extracts of *Camellia* including *C. senensis* (green tea) and *C*. *assaimic*, extracts of licorice, sea whip, aloe vera, chamomile, and the like.

The phenolic compounds described herein can be tannins and derivatives thereof. Tannins can be found in many plant species. For example, the tea plant (*Camellia sinensis*) has a naturally high tannin content. Green tea leaves are a major plant source of tannins, as they not only contain the tannic and gallic acid groups, but also prodelphinidin, a proanthocyanidin. Tannins are also found in wine, particularly red wine as well as in grape skins and seeds. Pomegranates also contain a diverse array of tannins, particularly hydrolysable tannins.

Tannic acid is a common naturally derived tannin. Tannic acid, as a cross-linking agent, is similar in many properties to that of many fixatives often used in the preparation and formation of xenograft or allograft tissue implants, for instance glutaraldehyde fixatives. Moreover, tannic acid can interact with other connective tissue components as well as elastin, and thus can stabilize additional components of the targeted connective tissue in the disclosed processes, in addition to the elastin component.

### Biocompatible Composition

In general, the phenolic compounds described herein can be provided as a biocompatible composition. In one embodiment, the phenolic compounds are used to stabilize connective tissue *in vivo.* Accordingly, in such embodiments, biocompatibility and cytotoxicity of the agents can be of importance in preparation of therapeutics including the disclosed compounds. At one time, tannic acid-containing preparations were suspected of causing hepatoxicity. This toxicity has since been primarily attributed to poor purity of the preparations and the inclusion of toxic gallic acid residues in the compositions. Accordingly, in one embodiment, the compositions include high purity tannic acid, with little or no free gallic acid residue included in the compositions. For example, in one embodiment, the compositions can comprise no more than about 5% free gallic acid residue in the preparation. In one embodiment, the compositions can comprise between about 1% and about 5% free gallic acid residue in the composition. A person or ordinary skill in the art will recognize that additional ranges of gallic free acid residues within the explicit ranges above are contemplated and are within the present disclosure.

In one embodiment, the compositions comprise an effective amount of pentagalloylglucose (PGG). PGG, which is the portion of a tannic acid molecule, including the hydrophobic core of tannic acid as well as multiple phenolic hydroxy groups, but does not posses the outer gallic acid residues and the hydrolyzable ester bonds associated with tannic acid. Thus, the possibility of release of free gallic acid residues over the course of a long-term application process can be prevented through utilization of a compound having no gallic acid residues, such as PGG, as the selected agent.

Compositions disclosed herein can include one or more phenolic compounds in a concentration that can vary over a selected range, with a concentration generally depending on the particular application, the delivery site targeted by the phenolic compound and the mode that will be used in the delivery process. For example, in one embodiment, a composition can include one or more phenolic compounds at a concentration from about 0.0001% to about 10%. It should be noted, however, that while these exemplary concentrations are effective in certain embodiments, compositions used herein comprises a wider range of phenolic compound concentrations. For example, actual concentrations used may be influenced by the organ targeted by the procedure, size of the targeted area, desired incubation time, and preferred pH, in addition to delivery mode, as mentioned above. In one embodiment, the disclosed compositions can include concentrations of a phenolic compound ranging from about 0.01% to about 2% and in additional embodiments from about 0.1% to about 1 %. A person of ordinary skill in the art will recognize that additional ranges within the explicit ranges above are contemplated and are within the present disclosure.

### Delivery System

In some embodiments, the exemplary method can comprise use of timed release or sustained release delivery systems. Such systems can be desirable, for instance, in situations where long term delivery of the agents to a particular organ or vascular location is desired. According to this particular embodiment, a sustained-release matrix can include a matrix made of materials, usually polymers, which are degradable by enzymatic or acid/base hydrolysis or by dissolution. Once located at or near the target tissue, e.g., inserted into the body, for instance in the form of a patch or a stent such as those further described below, such a matrix can be acted upon by enzymes and body fluids. The sustained-release matrix can be chosen from biocompatible materials such as liposomes, polylactides (polylactic acid), polyglycolide (polymer of glycolic acid), polylactide co-glycolide (co-polymers of lactic acid and glycolic acid) polyanhydrides, poly(ortho)esters, polyproteins, hyaluronic acid, collagen, chondroitin sulfate, carboxylic acids, fatty acids, phospholipids, polysaccharides, nucleic acids, polyamino acids, amino acids such as phenylalanine, tyrosine, isoleucine, polynucleotides, polyvinyl propylene, polyvinylpyrrolidone and silicone. Possible biodegradable polymers and their use are described, for example, in detail in Brem et al. (1991, J. Neurosurg. 74:441-6).

The dosage of the disclosed treatment agents can depend on the disease state or particular condition being treated and other clinical factors such as condition of the patient and the size and design of the device. In addition, the disclosed treatment agents can be administered in conjunction with other forms of therapy, e.g., surgical endovascular stent graft repair or replacement of an excessively damaged area of vasculature. For example, the aneurysm can be treated with the exemplary method described herein to arrest the aneurysm disease process. A currently utilized endovascular stent graft can be implanted after the treatment. The combination of the two treatments can reduce the risk of future expansion of the aneurysm and risk of graft migration at the aneurysm neck. For embodiments using the devices disclosed herein for delivery, a composition comprising one or more phenolic compounds can be targeted to a specific site, such as to a diagnosed aneurysm *in vivo,* using a less invasive procedure to provide delivery of the treatment agent locally from a biocompatible implantable device. Percutaneous entry into blood vessels and corresponding delivery technologies for catheter use for vascular procedures suitable for introducing the devices described herein are generally known to those of skill in the art, and can be adapted for use with the devices described herein.

The composition can be loaded in a drug delivery vehicle via encapsulation, coating, infusion, or any other loading mechanism, such as those known in the art. Prolonged absorption of a pharmaceutical form may be brought about by the inclusion of agents, such as aluminum monostearate and gelatin, which can delay absorption. For example, injectable depot forms can be made by forming microencapsule matrices including the elastin stabilization agent loaded in the matrix formed of biodegradable polymers such as polylactide-polyglycolide, poly(orthoesters) and poly(anhydrides). Depending upon the ratio of therapeutic agent to polymer and the nature of the particular polymer employed, the rate of drug release can be controlled. Depot injectable formulations can also be prepared by entrapping the therapeutic agents in liposomes or microemulsions which are compatible with body tissues. Therapeutic formulations may be sterilized, for example, by filtration through a bacterial-retaining filter or by incorporating sterilizing agents in the form of sterile solid compositions which can be dissolved or dispersed in sterile water or other sterile injectable media just prior to use. Endovascular drug delivery methods are described generally, for example, by DiCarlo, et al. (U.S. Patent No. 6,929,626), which describes an intraluminally placeable tubular device that can be located within the lumen of a blood vessel and coated or otherwise loaded with a drug, e.g., the phenolic compounds described herein.

Delivery of the therapeutic agent using the devices described herein can be supplemented with the use of a stent, which may or may not further comprise a drug coating. U.S. Patent 6,979,347 to Wu, et al., describes an apparatus and associated method for delivering a therapeutic substance through a coating on a stent, such as the phenolic compounds. For example, the phenolic compound, or a composition thereof, can be deposited into grooves on a stent using conventional spray or modified dip techniques.

In one embodiment, the disclosed agents can be targeted to connective tissue by use of a hydrogel delivery vehicle. Hydrogels are herein defined to include polymeric matrices that can be highly hydrated while maintaining structural stability. Suitable hydrogel matrices can include un-crosslinked and crosslinked hydrogels. In addition, crosslinked hydrogel delivery vehicles can optionally include hydrolyzable portions, such that the matrix can be degradable when utilized in an aqueous environment, e.g., *in vivo.* For example, the delivery vehicle can include a cross-linked hydrogel including a hydrolyzable cross-linking agent, such as polylactic acid, and can be degradable *in vivo.*

Hydrogel delivery vehicles can include, for example, natural polymers such as glycosaminoglycans, polysaccharides, proteins, and the like, as well as synthetic polymers, as are generally known in the art. A non-limiting list of hydrophilic polymeric materials that can be utilized in forming hydrogels can include dextran, hyaluronic acid, chitin, heparin, collagen, elastin, keratin, albumin, polymers and copolymers of lactic acid, glycolic acid, carboxymethyl cellulose, polyacrylates, polymethacrylates, epoxides, silicones, polyols such as polypropylene glycol, polyvinyl alcohol and polyethylene glycol and their derivatives, alginates such as sodium alginate or crosslinked alginate gum, polycaprolactone, polyanhydride, pectin, gelatin, crosslinked proteins peptides and polysaccharides, and the like.

Hydrogels are hydrophilic polymers that do not dissolve in aqueous solution generally as a result of crosslinking. Pluronic™ polymers generally comprise polyoxypropylene/polyoxyethylene block copolymers. Thus, hydrogels from the crosslinking of these block copolymers and similar compositions can be referred to as Pluronic™ hydrogels. Similarly, other hydrogels suitable for introduction into a patient can be similarly used. Pluronic™ hydrogels suitable for medical applications are described further, for example, in published PCT applications WO 01/41735A to Shah et al., entitled "Thermosensitive Biodegradeable Hydrogels Based on Low Molecular Weight Pluronics," and WO 2007/064152A to Han et al., entitled "Injectable Thermosensitive Pluronic Hydrogels Coupled With Bioactive Materials for Tissue Regeneration and Preparation Methods Thereof".

Polymeric particles for drug delivery generally include, for example, biocompatible polymers and may or may not be spherical. The polymeric particles generally have an average particle diameter of no more than about 5 microns, in further embodiments no more than a micron and in additional embodiments no more than about 250 nanometers, where the diameter is an average dimension through the particle center for non-spherical particles. The delivery of drugs using nanoparticles and microparticles is described further in published U.S. Patent application 2006/0034925 to Au et al, entitled "Tumor Targeting Drug-Loaded Particles". The formation of nanoparticles from poly(lactic-co-glycolic acid (PLGA) is described further in the examples below.

PGG formulations have been shown to form a gel under certain conditions. The conditions, such as concentration, during formation of the gel influence the resulting gel properties. In some embodiments, the PGG gel can be formulated to dissolve around 37 °C, the body temperature of a patient. Additionally or alternatively, PGG can be formulated as a gel that remains its gel form at around 37 °C or higher temperatures. The gel forms PGG can be used as drug delivery vehicle, for example, a slow release delivery vehicle, with properties adjusted as desired. The gel form PGG can also be used in combination with other delivery systems such as hydrogel and/or poly(lactic-co-glycolic acid) nanoparticles to provide release profiles for short or extended period (28 days).

The delivery of elastin stabilizing agents using a delivery vehicle, such as for delayed release, is described further in U.S. patent application publication number 2009/0214654 A1 to Isenburg et al., entitled "Treatment of Aneurysm With Application of Elastin Stabilizing Agent Embedded in a Delivery System".

Treatment with an elastin stabilizing agent can be combined with mechanical stabilization. In particular, a perivascular girdle wrap can be placed over the exterior of the aneurysm to provide mechanical stabilization along with the chemical stabilization. In some embodiments, the exemplary delivery systems described herein can be associated with a perivascular girdle wrap. For example, the delivery systems can be coated along the interior of the wrap and/or embedded in the material of the wrap. The wrap provides a close contact to the aneurysm site for consistent drug release. In these exemplary embodiments, the girdle wrap physically strengthens the vasculature at the aneurysm site to prevent it from bursting while the elastin stabilizing agents can act to stabilize and strengthen the tissue of the vessel along with inhibiting further degradation of the vessel at the location. The wrap can be formed from biocompatible polymers, such as polyesters, that can be formed into woven or non-woven fabrics.

## Claims

1. A device (900) for treating an isolated volume (934) in a blood vessel (930), the device (900) comprising:
a shaft (902) comprising a proximal end, a distal end and at least one lumen (922) extending from at or near the proximal end to at or near the distal end, wherein the lumen (922) connects with a port at its proximal end; and
a sealing element (904) comprising extendable distal and proximal balloons (906, 908) and a fluid exchange portion (970) that is on the shaft (902) of the device (900) comprising one or more exchange ports disposed on a side of one of the distal and proximal balloons (906, 908) in fluid communication with the lumen (922) of the shaft (902),
a flow device (124) of a therapeutic composition connected to an isolated volume (934) through a lumen (922), for delivering the therapeutic composition comprising tannins or a derivative thereof,
wherein the distal and proximal balloons (906, 908) each has a lower profile configuration and an extended configuration having a shape that pushes against the wall of the vessel (912) to form the isolated volume (934) within the vessel (912) with the exchange port of the fluid exchange portion (970) configured for the exchange of fluid between the isolated volume (934) and the lumen (922),
wherein the sealing element (904) in the extended configuration additionally comprises a bypass channel (912) to allow blood flow past the isolated volume (934) when the distal and proximal balloons (906, 908) contact the vessel wall (932).

2. The device (900) of claim 1 wherein the shaft (902) further comprises a rapid exchange guidewire lumen with a port at the distal end of the shaft (902).

3. The device (900) of claim 1 wherein the one or more exchange ports further comprises
a tubular conduit displaced from the shaft (902) having an opening (914) that is in fluid communication with the isolated volume (934).

4. The device (900) of any one of claims 1 to 3 wherein the fluid exchange portion (970) comprises a side channel (910) that is a portion of the lumen (922) having an opening (914) on the side of the sealing element (904) of the device (900), wherein the lumen (922) and the side channel (910) have an optional micro catheter (940) placed inside to go through the opening (914) on the side of the sealing element (904) to access the isolated volume (934).

5. The device (900) of any one of claims 1 to 4 wherein the exchange portion (970) comprise a liquid permeable structure.

6. The device (900) of any one of claims 1 to 5 further comprises an aspiration apparatus operably connected to the port to one of the lumens of the shaft (902).

7. The device (900) of any one of claims 1 to 6 further comprising a delivery element operably connected to the port to one of the lumens of the shaft wherein the delivery element comprises a stabilizing liquid that reacts with vessel tissue to stabilize the tissue.

8. The device (900) of any one of claims 1 to 7 wherein the tannins comprise pentagalloylglucose or a derivative of pentagalloylglucose.

9. The device (900) of any one of claims 1 to 8 wherein the lumen (924) is configured to provide fluid to extend one or more of the distal and proximal balloons (906, 908).

10. The device (900) of any one of claims 1 to 9 wherein the lumen (922) is configured to slowly leak the therapeutic composition into the isolated volume (934).

## Patentansprüche

1. Vorrichtung (900) zur Behandlung eines isolierten Volumens (934) in einem Blutgefäß (930), wobei die Vorrichtung (900) umfasst:
eine Welle (902), die ein proximales Ende, ein distales Ende und wenigstens ein Lumen (922) umfasst, das sich vom proximalen Ende oder in dessen Nähe bis zum distalen Ende oder dessen Nähe erstreckt, wobei das Lumen (922) an seinem proximalen Ende mit einem Anschluss verbunden ist; und
ein Abdichtungselement (904), das einen distalen und einen proximalen dehnbaren Ballon (906, 908) und ein Flüssigkeitsaustauschteil (970), das sich auf der Welle (902) der Vorrichtung (900) befindet, umfasst und einen oder mehrere Austauschanschlüsse, die sich auf einer Seite entweder des distalen oder des proximalen Ballons (906, 908) in Fluidkommunikation mit dem Lumen (922) der Welle (902) befinden;
eine Durchflussvorrichtung (124) für eine therapeutische Zusammensetzung, die über ein Lumen (922) mit einem isolierten Volumen (934) verbunden ist, zur Abgabe der therapeutischen Zusammensetzung, die Tannine oder ein Derivat davon umfasst;
wobei der distale und der proximale Ballon (906, 908) jeweils Konfiguration mit einem niedrigeren Profil und eine gedehnte Konfiguration mit einer Form, die gegen die Wand des Gefäßes (912) drückt, aufweist, wobei das isolierte Volumen (934)innerhalb des Gefäßes (912) entsteht, wobei der Austauschanschluss des Flüssigkeitsaustauschteils (970) für den Austausch von Flüssigkeit zwischen dem isolierten Volumen (934) und dem Lumen (922) konfiguriert ist, wobei das Abdichtungselement (904) in der gedehnten Konfiguration zusätzlich einen Umleitungskanal (912) umfasst, um zu ermöglichen, dass Blut an dem isolierten Volumen (934) vorbei fließt, wenn der distale und der proximale Ballon (906, 908) mit der Gefäßwand (932) in Kontakt stehen.

2. Vorrichtung (900) gemäß Anspruch 1, wobei die Welle (902) weiterhin ein Führungsdrahtlumen für schnellen Austausch mit einem Anschluss am distalen Ende der Welle (902) umfasst.

3. Vorrichtung (900) gemäß Anspruch 1, wobei der eine oder die mehreren Austauschanschlüsse weiterhin eine röhrenförmige Leitung umfassen, die gegenüber der Welle (902) verschoben ist und eine Öffnung (914) aufweist, die sich in Fluidkommunikation mit dem isolierten Volumen (934) befindet.

4. Vorrichtung (900) gemäß einem der Ansprüche 1 bis 3, wobei der Flüssigkeitsaustauschteil (970) einen Seitenkanal (910) umfasst, der ein Teil des Lumens (922) ist und auf der Seite des Abdichtungselements (904) der Vorrichtung (900) eine Öffnung (914) aufweist, wobei das Lumen (922) und der Seitenkanal (910) einen optionalen Mikrokatheter (940) aufweisen, der innerhalb platziert wird und auf der Seite des Abdichtungselements (904) durch die Öffnung (914) verläuft, um Zugang zu dem isolierten Volumen (934) zu erhalten.

5. Vorrichtung (900) gemäß einem der Ansprüche 1 bis 4, wobei der Austauschteil (970) eine flüssige durchlässige Struktur umfasst.

6. Vorrichtung (900) gemäß einem der Ansprüche 1 bis 5, die weiterhin eine Saugvorrichtung umfasst, die funktionell mit dem Anschluss an eines der Lumen der Welle (902) verbunden ist.

7. Vorrichtung (900) gemäß einem der Ansprüche 1 bis 6, die weiterhin ein Abgabeelement umfasst, das funktionell mit dem Anschluss an eines der Lumen der Welle verbunden ist, wobei das Abgabeelement eine stabilisierende Flüssigkeit umfasst, die mit Gefäßgewebe reagiert und dadurch das Gewebe stabilisiert.

8. Vorrichtung (900) gemäß einem der Ansprüche 1 bis 7, wobei die Tannine Pentagalloylglucose oder ein Derivat von Pentagalloylglucose umfassen.

9. Vorrichtung (900) gemäß einem der Ansprüche 1 bis 8, wobei das Lumen (924) so konfiguriert ist, dass es Flüssigkeit bereitstellt, um einen oder mehrere der distalen und proximalen Ballons (906, 908) auszudehnen.

10. Vorrichtung (900) gemäß einem der Ansprüche 1 bis 9, wobei das Lumen (922) so konfiguriert ist, dass es die therapeutische Zusammensetzung langsam in das isolierte Volumen (934) auslaufen lässt.

## Revendications

1. Dispositif (900) pour traiter un volume isolé (934) dans un vaisseau sanguin (930), le dispositif (900) comprenant :
- un corps (902) comprenant une extrémité proximale, une extrémité distale et au moins une lumière (922) s'étendant depuis le niveau de l'extrémité proximale ou à proximité de celle-ci jusqu'au niveau de l'extrémité distale ou à proximité de celle-ci, dans lequel la lumière (922) communique avec un orifice au niveau de son extrémité proximale ; et
- un élément d'étanchéité (904) comprenant des ballonnets distal et proximal (906, 908) déployables et une partie d'échange de fluide (970) qui est sur le corps (902) du dispositif (900), comprenant un ou plusieurs orifices d'échange disposés sur un côté d'un des ballonnets distal et proximal (906, 908) en communication fluide avec la lumière (922) du corps (902) ;
- un dispositif d'écoulement (124) d'une composition thérapeutique connecté à un volume isolé (934) par l'intermédiaire d'une lumière (922), pour délivrer la composition thérapeutique comprenant des tanins ou un dérivé de ceux-ci,
dans lequel les ballonnets distal et proximal (906, 908) ont chacun une configuration plus plate et une configuration déployée ayant une forme qui pousse contre la paroi du vaisseau (912) pour former le volume isolé (934) à l'intérieur du vaisseau (912), avec l'orifice d'échange de la partie d'échange de fluide (970) configuré pour l'échange de fluide entre le volume isolé (934) et la lumière (922),
dans lequel l'élément d'étanchéité (904) dans la configuration déployée comprend en outre un canal de dérivation (912) pour permettre à l'écoulement sanguin de franchir le volume isolé (934) lorsque les ballonnets distal et proximal (906, 908) sont en contact avec la paroi du vaisseau (932).

2. Dispositif (900) selon la revendication 1, dans lequel le corps (902) comprend en outre une lumière de fil-guide à échange rapide avec un orifice au niveau de l'extrémité distale du corps (902).

3. Dispositif (900) selon la revendication 1, dans lequel les un ou plusieurs orifices d'échange comprennent en outre un conduit tubulaire dissocié du corps (902) ayant une ouverture (914) qui est en communication fluide avec le volume isolé (934).

4. Dispositif (900) selon l'une quelconque des revendications 1 à 3, dans lequel la partie d'échange de fluide (970) comprend un canal latéral (910) qui est une partie de la lumière (922) ayant une ouverture (914) sur le côté de l'élément d'étanchéité (904) du dispositif (900), dans lequel la lumière (922) et le canal latéral (910) ont un micro-cathéter (940) facultatif placé à l'intérieur pour passer à travers l'ouverture (914) sur le côté de l'élément d'étanchéité (904) pour accéder au volume isolé (934).

5. Dispositif (900) selon l'une quelconque des revendications 1 à 4, dans lequel la partie d'échange (970) comprend une structure perméable aux liquides.

6. Dispositif (900) selon l'une quelconque des revendications 1 à 5, comprenant en outre un appareil d'aspiration fonctionnellement connecté à l'orifice de l'une des lumières du corps (902).

7. Dispositif (900) selon l'une quelconque des revendications 1 à 6, comprenant en outre un élément de délivrance fonctionnellement connecté à l'orifice de l'une des lumières du corps, dans lequel l'élément de délivrance comprend un liquide stabilisateur qui réagit avec les tissus du vaisseau pour stabiliser les tissus.

8. Dispositif (900) selon l'une quelconque des revendications 1 à 7, dans lequel les tanins comprennent le pentagalloylglucose ou un dérivé de pentagalloylglucose.

9. Dispositif (900) selon l'une quelconque des revendications 1 à 8, dans lequel la lumière (924) est configurée pour fournir un fluide pour déployer un ou plusieurs des ballonnets distal et proximal (906, 908).

10. Dispositif (900) selon l'une quelconque des revendications 1 à 9, dans lequel la lumière (922) est configurée pour laisser la composition thérapeutique s'infiltrer lentement dans le volume isolé (934).
